# EUROPEAN PATENT SPECIFICATION

(11) **EP 2 318 082 B1**
(45) Date of publication and mention of the grant of the patent: **30.07.2014**
(21) Application number: 09804399.5
(22) Date of filing: 10.08.2009
(51) Int. Cl.: A61M 25/00

(54) **REVERSE CATHETER**
UMGEKEHRTER KATHETER
CATHÉTER INVERSE

(30) Priority: 08.08.2008 US 87177 P; 24.02.2009 US 155149 P
(43) Date of publication of application: 11.05.2011
(73) Proprietor: Osprey Medical Inc., St. Paul, MN 55112 (US)
(72) Inventor: BILNEY, Adam, Lucas, Melbourne, Victoria 8008 (AU); KAYE, David, Martin, Beaumaris, Victoria 3016 (AU); POWER, John, Melmouth, Williamstown, Victoria 3016 (AU); BYRNE, Melissa, Albert Park, Victoria 3206 (AU); CHRISTOV, Steve, West Coburg, Victoria 3058 (AU)
(74) Representative: Kransell & Wennborg KB
(86) International application number: PCT/AU2009/001021
(87) International publication number: WO 2010/015044

(56) References cited:
- WO-A1-00/12169
- WO-A1-00/66019
- WO-A1-03/080166
- WO-A1-2003/080166
- WO-A1-2007/022592
- WO-A2-03/097122
- US-A- 4 867 742
- US-A- 5 222 971
- US-A- 5 441 516
- US-A- 5 456 667
- US-A1- 2006 015 065
- US-A1- 2008 082 050

## Description

### Field of the Invention

The present invention relates to improvements in devices designed to circulate a perfusate into and/or out of a portion of a body.

### Background of the Invention

Many therapeutic procedures can be performed percutaneously. Such procedures include investigative and diagnostic methods as well as medical and surgical treatments. Percutaneous techniques are advantageous because they are minimally invasive, and therefore present a reduced risk to the patient when compared with invasive surgeries requiring open access to tissues within the patient's body.

Catheters and sheaths are typically used to access internal sites by entering the patient's body through an incision or other entry point to the peripheral vasculature and navigating through the torturous paths of the vascular system to the target site. Tools, cameras illumination sources and transducers can be deployed through the catheters and sheaths enabling a physician to inspect the site, take biopsies, implant devices, repair damage and perform numerous other tasks often on an outpatient basis.

Minimally invasive percutaneous approaches have also presented an opportunity for localized delivery of therapeutic agents. Localization facilitates improved treatment since the agent may be delivered directly to a target site, (for example, an organ) thereby avoiding "first pass" degradation in the liver. The agent may therefore exhibit an improved therapeutic effect when compared with the same agent administered orally, intravenously or via the intramuscular route. Moreover, where the therapeutic agent has benefit to the target site but is likely to be toxic to other organs or tissues, localized delivery can minimize the exposure and hence the risk presented to cells in other parts of the body. An example is where a cytotoxic drug is to be delivered to a cancerous organ, but it is desired to minimize or prevent exposure of healthy organs to the drug.

Various devices and methods to facilitate the percutaneous delivery and collection of fluids to organs and tissues of the human body have been described in the art. Typically, these devices and methods include a first catheter to deliver a fluid to an organ or tissue and a second catheter to collect fluid exiting the organ or tissue. The collected fluid may be returned to the organ, or discarded as waste outside the body. These devices and methods have had varying degrees of success and degrees of invasiveness. Successful applications have been developed for use with cardiac tissue, but the conventional devices and techniques used for cardiac treatment are not always well suited for areas outside the heart. Some reasons for this are that vessels come in different sizes. Therefore an apparatus used in the heart is not generally suitable for applications outside the heart. Additionally, the pathways of the heart usually require special consideration, such as manoeuvrability around the arches of the coronary system.

Methods of catheter based isolated limb perfusion or infusion generally require standard catheters of greater than 50 cm length to be introduced from the contralateral femoral artery, across the aortic bifurcation and advanced to the common femoral artery (CFA). This ensures that the outlet of the catheter is proximal to the branch of the profunda femoris artery (PFA). In patients with peripheral vascular disease (PVD) or an occluded superficial femoral vein, often the PFA provides the majority of blood flow to the lower limb. Therefore placing the outlet of the cannula in the CFA is ideal when the whole limb needs to be perfused.

Standard catheter introducers placed in the groin will have the outlet of the introducer in the superficial femoral artery (SFA), if inserted in an antegrade direction. Therefore, a standard balloon catheter would only deliver perfusate downstream of the SFA, which is not optimal. Accordingly, there exists a need to provide an improved device, system and method that in certain situations will permit more optimal delivery or drainage of a perfusate to a selected area of the body.

US2008/066019 discloses systems and methods for treating a vessel. It include devices having a main elongated element with a balloon at its distal end, an auxiliary elongated element wherein a distal end of the auxiliary elongated element is proximal to the balloon, and a core wire having a internal core wire portion and an external core wire portion, wherein the external core wire portion is external to the balloon. Inflation of the balloon causes guidewires positioned within the device and external core wires to be pushed up against a lesion in the vessel, providing a focused force for cracking the lesion.

WO2003/097122 discloses an apparatus for treating an occlusive region of a blood vessel. It includes a catheter including a first lumen extending from an end port to a first side port, and a second lumen extending from a proximal end of the catheter to a second side port. A balloon is mounted on the catheter between the first and second side ports. During use, a guidewire is placed into an occlusive region, and the guidewire is backloaded through the first and second lumens. The catheter is advanced over the guidewire, and the balloon is expanded to isolate the occlusive region. The guidewire is removed, and an agitator is advanced through the second lumen. After the agitator is agitated to dislodge occlusive material from the occlusive region, the agitator is removed, and the loose occlusive material is aspirated via the second side port.

WO2003/081066 discloses a venous port for withdrawal of fluid in which one or more extensions are provided which can selectively extend to remove impediments from an aperture of the port.

The reference to any prior art in this specification is not, and should not be taken as an acknowledgement or any form of suggestion that the referenced prior art forms part of the common general knowledge in Australia.

### Summary

The present invention in one preferred aspect provides for a catheter for conducting a fluid into or out of a blood vessel, the catheter comprising a body having a proximal end, a closed distal end and a mid-longitudinal axis; and a balloon attached to the body, the balloon having a proximal portion and a distal portion. The body has a first lumen in communication with the balloon and a second lumen adapted to convey a fluid to or from the blood vessel through a side opening in the body, the side opening being located adjacent the proximal portion of the balloon.

In another preferred aspect, the present invention provides for a system for conducting a fluid into or out of a blood vessel, the system comprising a catheter including a body having a proximal end, a distal end and a mid-longitudinal axis; a balloon attached to the body, the balloon having a proximal portion and a distal portion. The body has a first lumen in communication with the balloon, a second lumen having a length and an opening proximate the distal end, and a third lumen extending between a connection port and a side opening located adjacent the proximal portion of the balloon. The third lumen is adapted to convey a fluid to or from the port and the blood vessel through the side opening. The system further includes an instrument insertable through the second lumen, the instrument having a length greater than the length of the second lumen.

In another case, there is a method for removing a perfusate from an individual, comprising placing a distal end of a catheter into a vein in an antegrade direction relative to the blood flow; diverting the blood flow from the vein through the catheter, the blood flow including the perfusate; and removing the perfusate from the individual using the catheter.

### Brief Description of the Figures

Fig. 1 is a partial perspective view of a reverse catheter in accordance with a preferred embodiment of the present invention.
Fig. 2 is a partial side elevation view of the catheter of Fig. 1.
Fig. 3 is a perspective view of the catheter of Fig. 1 shown inserted in an antegrade direction in a blood vessel.
Fig. 4 is a diagram showing a system utilising the catheter of Fig. 1 in accordance with another preferred embodiment of the present invention.
Fig. 5 is a perspective view of a reverse catheter in accordance with another preferred embodiment of the present invention.
Fig. 6 is a side elevation view of the catheter of Fig. 5.
Fig. 6A is a partial cross sectional side view of the catheter along line A-A of Fig. 6.
Figs. 7A shows a cover covering the catheter of Fig.1.
Figs. 7B and 7C show deployment of the catheter of Fig. 1 with the cover of Fig. 7A in accordance with a preferred embodiment of a method of the present invention.
Fig. 8 is a perspective view of the catheter of Fig. 1 shown inserted in a lower extremity vein, and the reverse catheter of Fig. 5 shown inserted a lower extremity artery.
Fig. 8A is an expanded view of the positioning of the catheter of Fig. 1 and the reverse catheter of Fig. 5 taken along line A-A of Fig. 8.
Fig. 9 is a partial perspective view of a reverse catheter in accordance with another preferred embodiment of the present invention.
Fig. 10 is a partial side view of the catheter of Fig. 9.
Fig. 11 is a perspective view of the catheter of Fig. 9 shown inserted in an antegrade direction in a blood vessel.
Fig. 12 is a perspective view of the catheter of Fig. 9 shown inserted in an antegrade direction in a blood vessel of an extremity wherein occlusion is effected by a tourniquet.
Fig. 13 is a partial side view of an occlusion catheter in accordance with another preferred embodiment of the present invention.
Fig. 14 is a partial perspective view of a reverse catheter in accordance with another preferred embodiment of the present invention.
Fig. 15 is a partial side view of the catheter of Fig. 14.
Fig. 16A is a perspective view of catheter of Fig. 14 shown inserted in an antegrade direction in a blood vessel of an extremity wherein occlusion is effected by a balloon catheter.
Fig. 16B is a perspective view of the catheter of Fig. 9 shown inserted in an antegrade direction in a blood vessel of an extremity wherein occlusion is effected by a balloon catheter.
Fig. 17A is a perspective view of the catheter of Fig. 1 shown inserted in a lower extremity vein, and a standard perfusion catheter inserted into a lower extremity artery from the contralateral side.
Fig. 17B is a perspective view of the catheter of Fig. 1 shown inserted in a lower extremity vein, and a standard perfusion catheter inserted into a lower extremity artery from the same side.

### Detailed Description of the Drawings

Alternative embodiments of the invention will be apparent to those skilled in the art from consideration of the specification and practice of the invention disclosed herein. It is intended that the specification and examples be considered as exemplary only, with a true scope of the invention being indicated by the claims which follow. Wherever possible, like numbers will refer to like parts.

As used herein, "fluid" is intended to mean a flowable material that may or may not include solid particles. The phrase "blood vessel" is intended to include an artery, vein, or capillary.

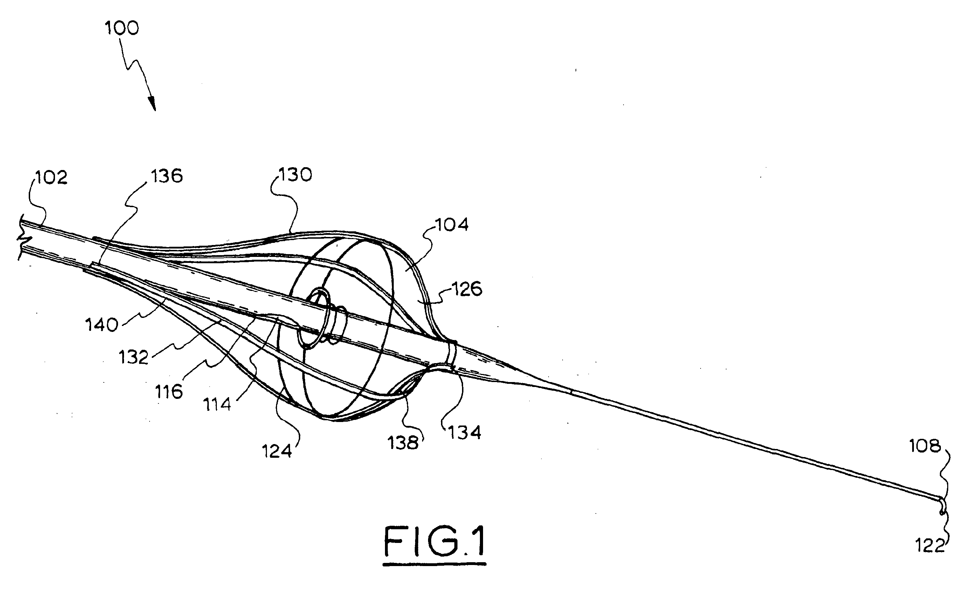

Figs. 1 to 3 show a preferred embodiment of a reverse catheter 100 having a body 102 and an occlusion means such as balloon 104. The preferred elements of catheter 100 and their interrelationship are described below.

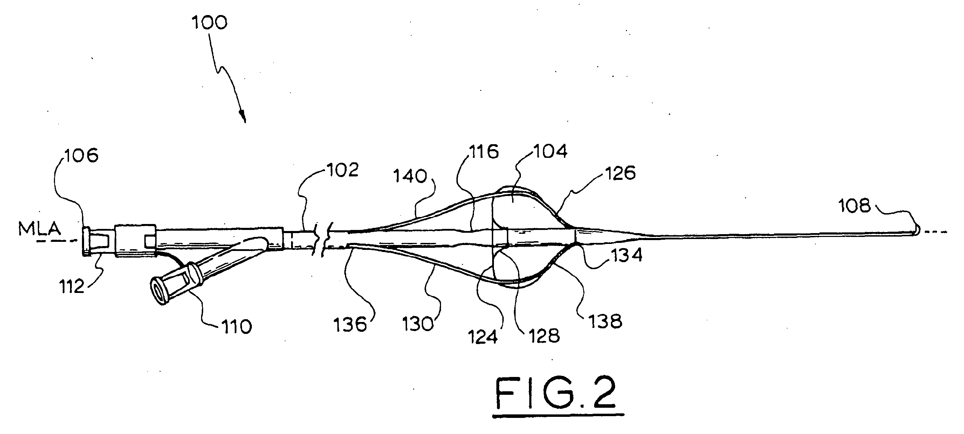

Referring to Figs. 1 and 2, body 102 includes a proximal end 106, a preferably closed distal end 108 and a mid-longitudinal axis MLA running through proximal end 106 and distal end 108. Proximal end 106 preferably includes a hub 110 configured as an inflation hub for balloon 104. Body 102 includes a lumen (not illustrated) extending between hub 110 and balloon 104 to inflate balloon 104. Body 102 preferably includes a second hub 112 adapted to connect to a source or repository of perfusion fluid. Body 102 includes a lumen 114 extending between hub 112 and a side opening 116 to convey perfusate between side opening 116 and hub 112. It will be appreciated that the reference to "side" is intended for easy reference and not limiting based on a particular position or orientation of the catheter in use.

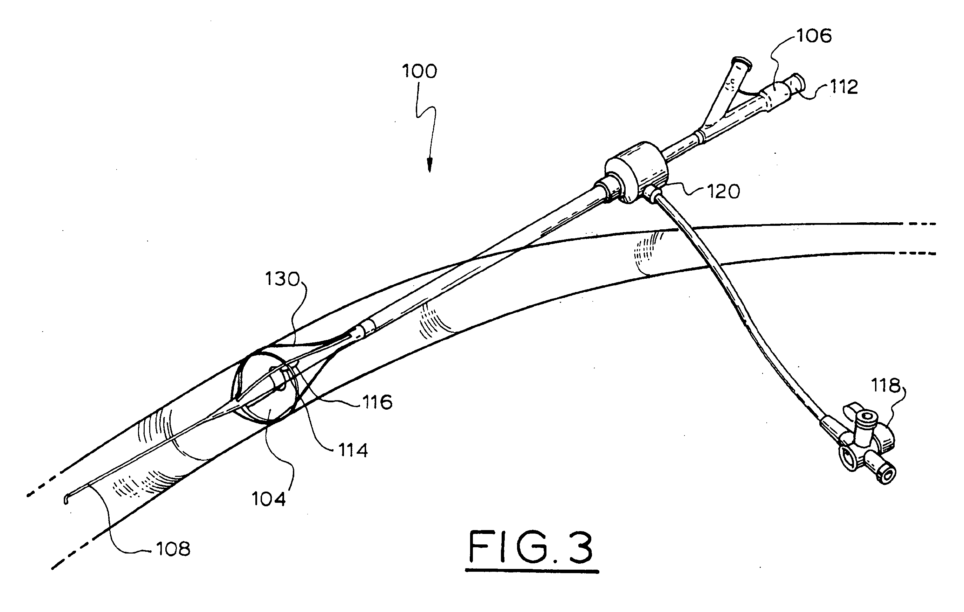

If desired, proximal end 106 may include hubs or ports for other uses. For example only, Fig. 3 shows a tap 118 connected to a hub 120 near proximal end 106.

As shown in Figs. 1 and 2, distal end 108 preferably includes an atraumatic tip 122 preferably configured as a J-tip or hook. It will be appreciated that tip 122 may be configured in a variety of ways without departing from the scope of the present invention. Examples of other tips are shown and described in International Application No. PCT/AU2006/001234 (Publication No. WO 2007/022592).

Referring to Figs. 2 and 3, balloon 104 is preferably located closer to distal end 108 than proximal end 106. Balloon 104 includes a proximal portion 124 and a distal portion 126. Proximal portion 124 and distal portion 126 are preferably asymmetrically shaped relative to one another so that distal portion 126 generally forms a dome shape while proximal portion 124 generally forms a flattened end. Proximal portion 124 is preferably attached to body 102 so as to form a funnel region 128 relative to the exterior surface of body 102. Side opening 116 is preferably located adjacent proximal portion 124 so that perfusate delivery or collection occurs proximally relative to balloon 104. It will be appreciated that the balloon may be sized as desired (e.g., long or short).

As shown in Figs. 1 and 2, body 102 preferably includes an expandable support structure 130. Support structure 130 is preferably adapted to support and/or dilate a blood vessel into which catheter 100 is inserted. In particular, support structure 130 is beneficial in supporting the wall of the blood vessel against collapse, especially when a negative pressure is present. Support structure 130 preferably includes a plurality of trusses 132 equally circumferentially spaced about the mid-longitudinal axis of body 102. Each of trusses 132 has a first end 134 and a second end 136 connected at different points along the length of body 102. Preferably, first end 134 is connected to body 102 distally of distal portion 126 of balloon 104, while second end 136 is connected proximally of side opening 116. Support structure 130 is preferably shaped so that a distal facing portion 138 generally forms a dome shape while a proximal facing portion 140 generally forms a cone shape with a gentle curve along a portion of its length as shown in Fig. 2. Examples of other forms of support structures are shown and described in International Application No. PCT/AU2005/000237 (Publication No. WO 2005/082440).

The location of balloon 104 within support structure 130 beneficially permits the balloon to perform more than one function. For example, in addition to occluding the vessel, balloon is well placed to control trusses 132. When balloon 104 is deployed, the expansion of balloon 104 moves trusses 132 to deploy support structure 130. Support structure 130 may be configured as desired so that deflation of balloon 104 collapses support structure. Trusses 132 may be configured to be of sufficient resiliency and strength to support tissue without the aid of balloon 104 if desired.

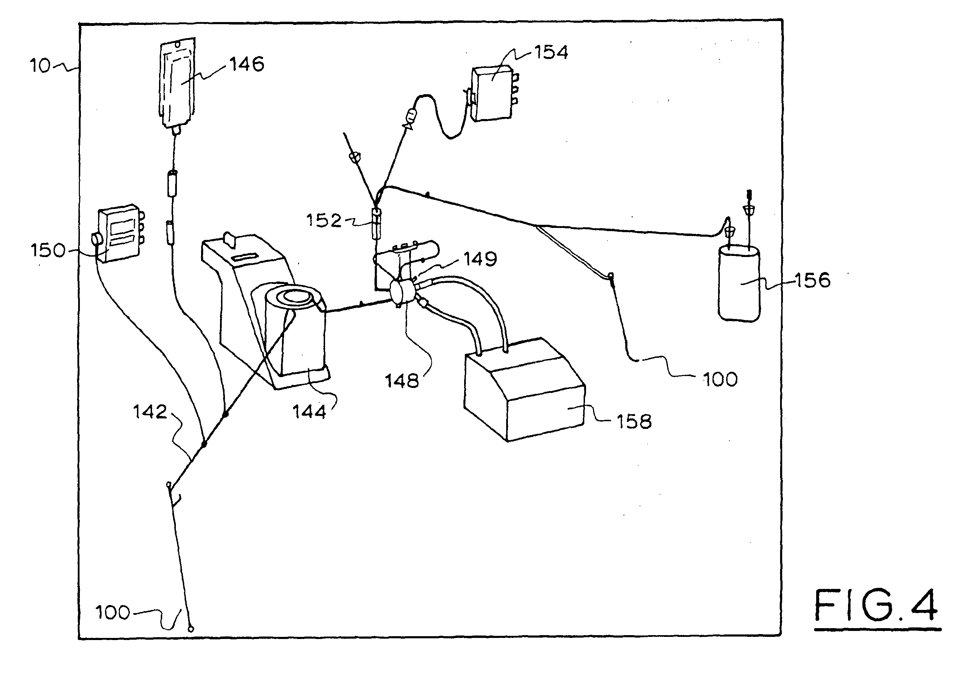

Fig. 4 shows an exemplary system 10 utilising catheter 100. System 10 preferably includes a venous collection catheter 100 connected to a tubing 142 at hub 112 (Fig. 2). Tubing 142 preferably runs through a peristaltic pump 144 configured to draw fluid into a perfusate reservoir 146 and/or oxygenator 148 (if the fluid is intended for re-use). Oxygenator preferably includes introducer ports 149 adapted for connection with other components either directly or indirectly via tubing. Pump 144 is preferably operationally connected to a pressure monitor 150 in order to permit the system to be automated. The system further preferably includes a pathway from pump 144 that leads to a bubble trap 152, a second pressure monitor 154, a collection bag 156 and/or delivery catheter 100. A heater 158 is preferably connected to oxygenator 148 to heat the fluid to a sufficient temperature for the intended purpose (e.g., introduction back into a patient). Various components of the system may preferably be in communication with a computer programmed to operate the system within specified parameters.

The catheter may be constructed from a variety of medically acceptable materials. For example, the trusses of support structure 130 may be made from Nitinol, or an elastic polymer (which may open up when the balloon is inflated).

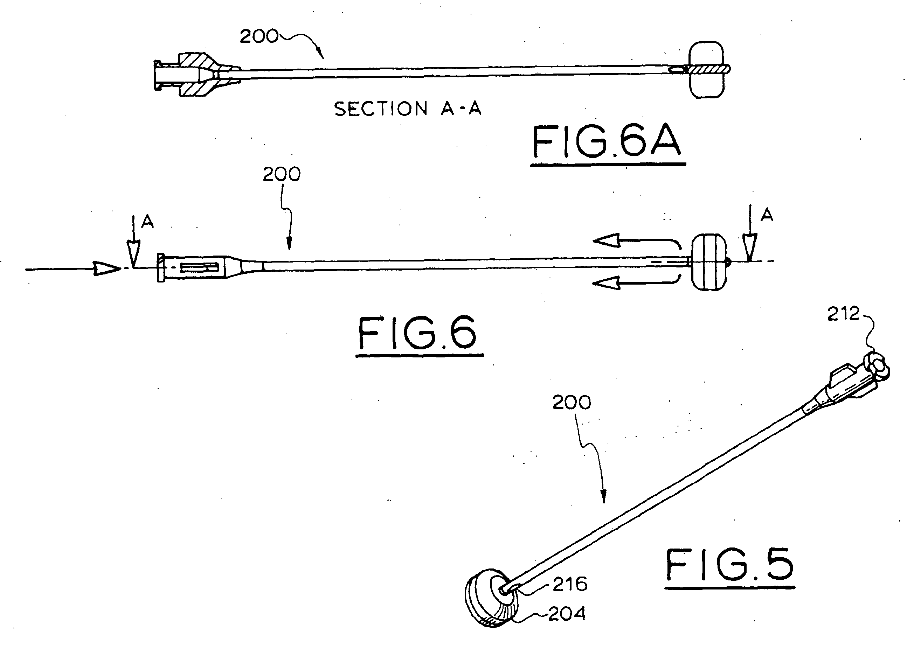

Referring now to Figs. 5 to 6A, a catheter 200 is shown in accordance with another preferred embodiment of the present invention. Catheter 200 is similar to catheter 100 except that the support structure is omitted around balloon 204. Additionally, the proximal and distal portions of balloon 204 are symmetrically shaped. Catheter 200 is preferably configured for use as a reverse delivery catheter and may be used in place of catheter 100 presently shown in Fig. 4. The black arrows in Fig. 6A represent the flow of fluid when catheter 200 is being used for the purpose of perfusion. It will be appreciated that catheter 100 may also be configured as a reverse delivery catheter as desired.

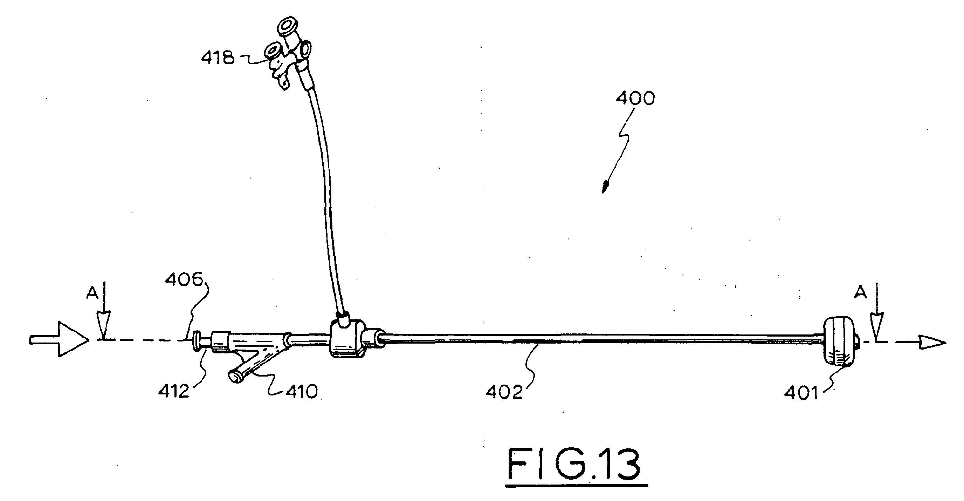

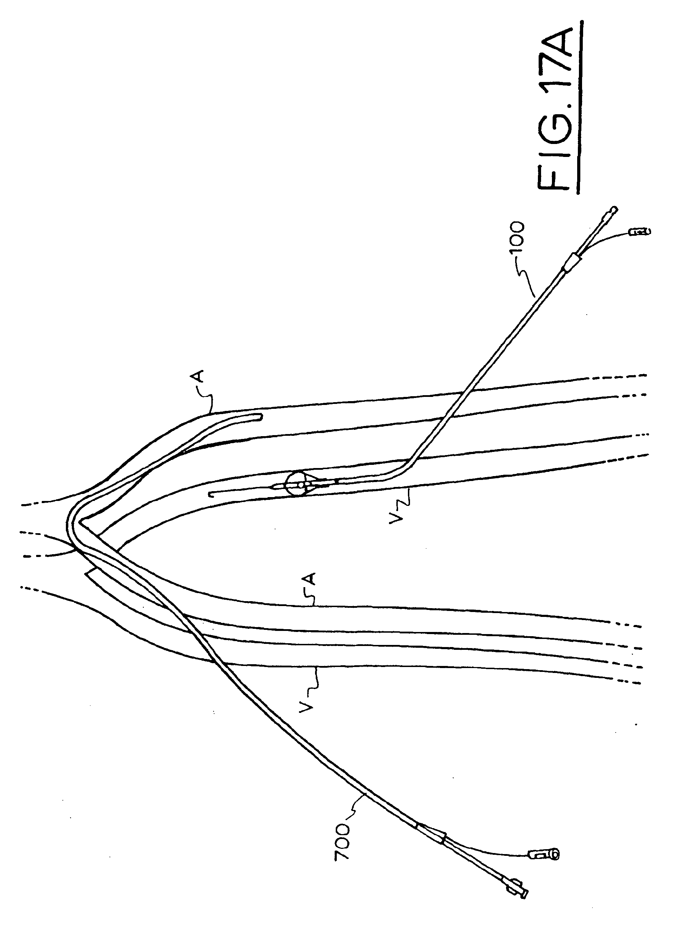

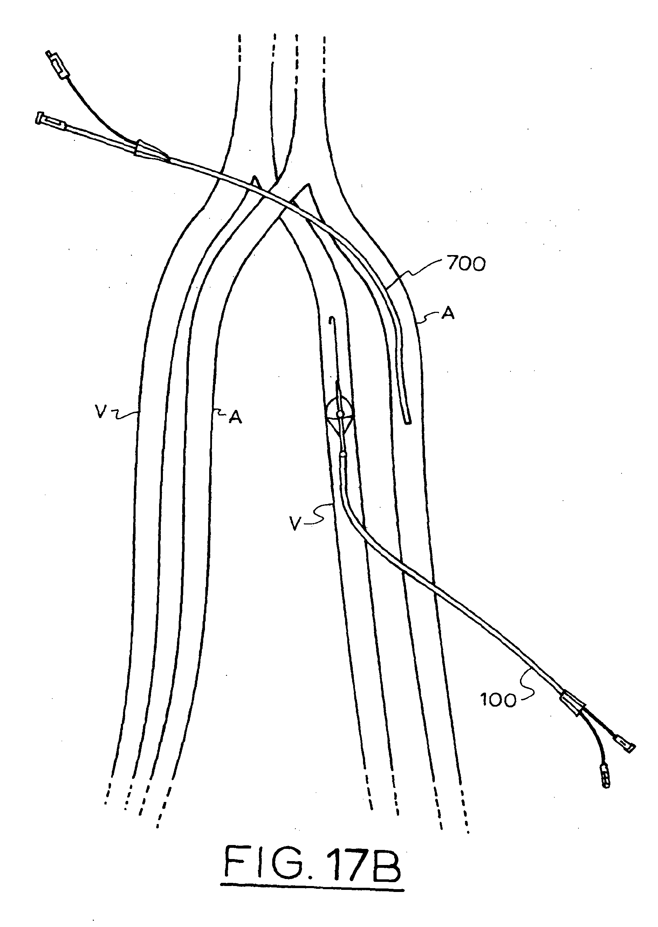

A delivery device of the kind illustrated in Figs. 5 to 6A is suitable for delivering a blood (and therapeutic agent) solution from the artificial flow path to the targeted tissues. (See, e.g., Figs. 13, 17A and 17B). The blood/therapeutic agent solution may then be collected after passing through the target tissue using a venous collection device, such as catheter 100 in Fig. 2. Collected solution can then be re-circulated through the targeted tissue with re-oxygenated collected blood using a system such as system 10 in Fig. 4. Such a perfusion method is beneficial as it maximizes the efficiency of the therapeutic agent by eliminating break down in the liver and stomach, and also reduces or eliminates the toxicity issues associated with specific treatments reaching non-target tissue. Re-circulation further improves the uptake of therapeutic agents by increasing the exposure time to the target tissue, improving the effectiveness of treatment.

Having described the preferred components of catheter 100 and catheter 200, a preferred method of use will now be described with reference to Figs. 3, 5, and 7A to 8A. To perfuse a selected area of the body, the medical practitioner inserts catheter 200 into an artery (A) in a retrograde direction relative to the blood flow. Balloon 204 is deployed to occlude the pathway. The perfusate is introduced into the blood stream through hub 212, through a lumen and side opening 216 and into the blood vessel.

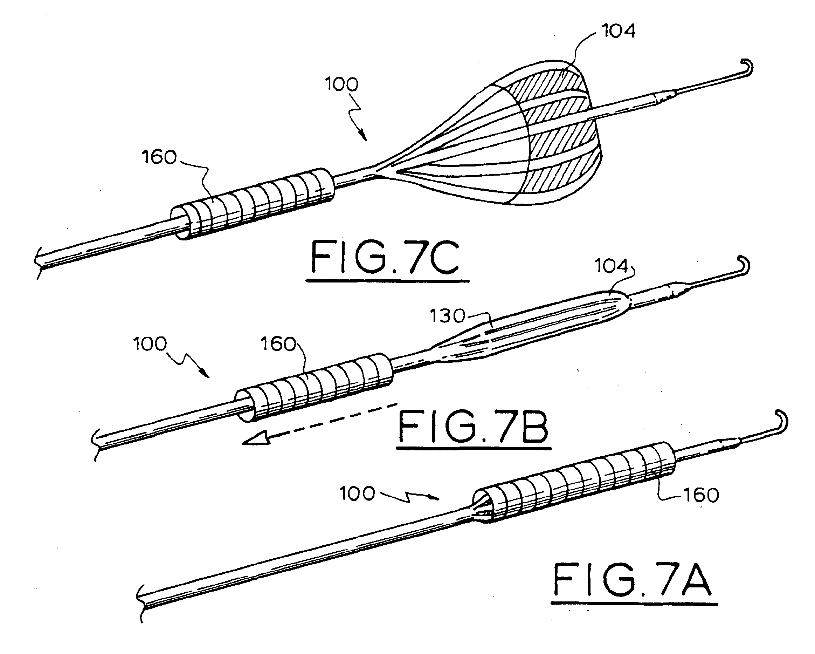

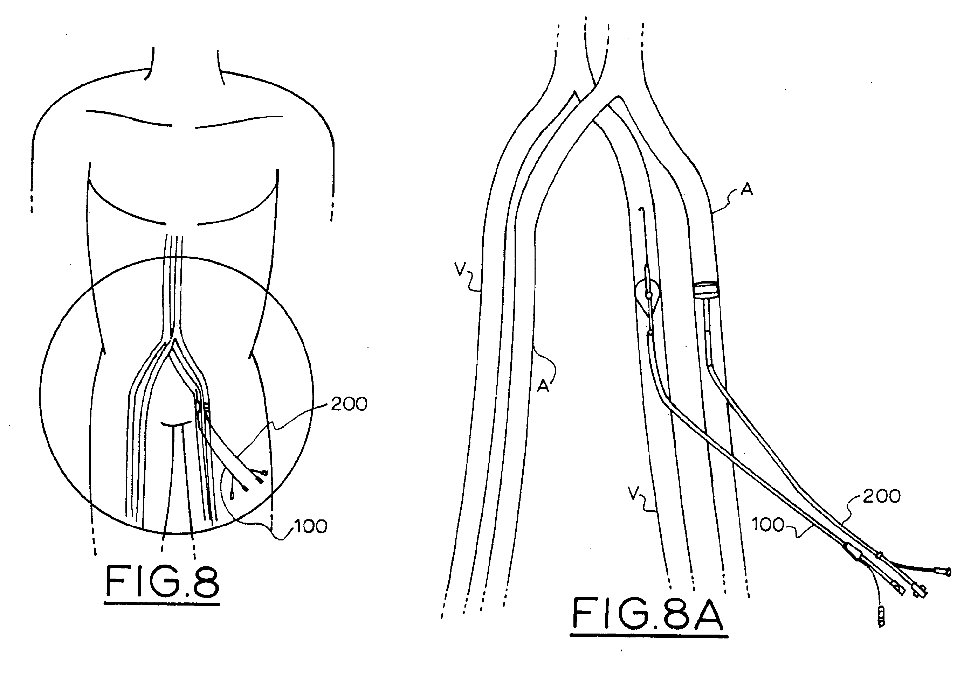

To collect the perfusate, the medical practitioner inserts catheter 100 into a vein (V) in an antegrade direction relative to the blood flow in an undeployed state, shown in Fig. 7A. A cover 160 is withdrawn to expose support structure 130 and balloon 104 as shown in Fig. 7B. Catheter 100 is moved to its deployed and engaged state as shown in Fig. 7C by deploying balloon 104 to occlude the pathway (see Fig. 8A). It will be appreciated that cover 160 need not cover the balloon. Support structure 130 may be deployed at the same time as balloon 104, or deployed prior to inflating the balloon as desired. The blood flow is diverted from the vein through side opening 116, lumen 114 and into a collection reservoir via hub 112 of catheter 100. Thereafter the blood containing the perfusate may be circulated into a delivery catheter for re-use as desired, or collected for later use or disposal. The perfusate being introduced into the delivery catheter may be replenished as needed and optionally supplemented by previously circulated perfusate. For example, system 10 shown in Fig. 4 may be used in performing the above method. Other exemplary circuits are described in International Application No. PCT/US2007/016369 (Publication No. WO 2008/011100).

As shown in Fig. 8 and 8A, the catheters are preferably positioned at similar points in the respective artery and vein. It will be appreciated that the positioning may be varied as desired. Additionally, the above system and method may be used on only a portion of a limb if desired, for example, after an amputation.

Once the procedure has been substantially completed, balloon 104 is deflated, support structure 130 is collapsed and cover 160 is moved substantially back to its original position over support structure 130. It will be appreciated that the procedure may be performed without cover 160. Where support structure 130 is configured to spring outward, cover 160 may function to contain support structure 130 so that as cover 160 is withdrawn, support structure 130 deploys due to its own resiliency. Cover 160 may be used to collapse support structure 130 if desired. Cover 160 provides several advantages. For example, cover 160 protects the balloon and elements of the support structure during packaging and movement of the catheter. Cover 160 also protects vessels during insertion and removal of the catheter.

To perfuse a lower limb, catheter 100 is inserted into the vessel of the limb where perfusate is to be collected or delivered. By inserting the catheter in an antegrade direction in a vein, or a retrograde direction if inserting the catheter into an artery, the need to traverse the femoral archway (and the associated technical complexity of such a procedure) is eliminated. This has many advantages, such as use of catheters requiring less materials (and therefore costs), and a procedure that is less invasive.

It will be appreciated that the steps described above may be performed in a different order, varied, or certain steps omitted entirely without departing from the scope of the present invention. For example, catheter 100 may be inserted and withdrawn without the use of cover 160 if desired. Catheter 100 may be used as a reverse delivery catheter in lieu of catheter 200.

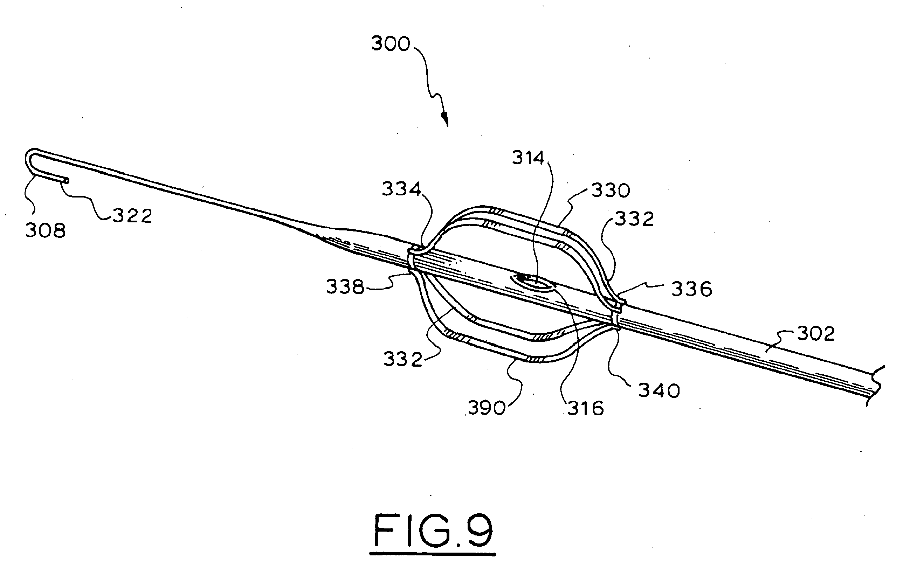

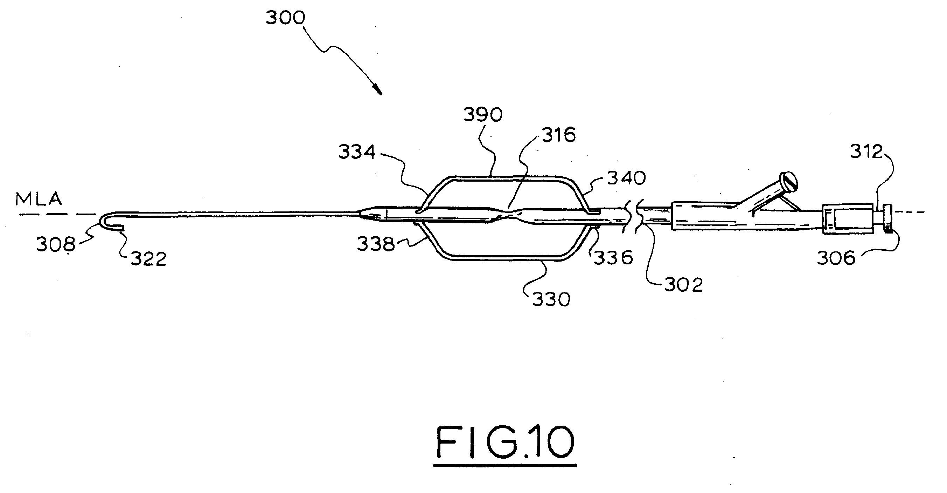

Referring now to Figs. 9 to 12, a catheter 300 is shown in accordance with another preferred embodiment of the present invention. Catheter 300 is similar to catheter 100 except that the balloon is omitted. Referring to Figs. 9 and 10, body 302 includes a proximal end 306, a preferably closed distal end 308 and a mid-longitudinal axis (MLA) running through proximal end 306 and distal end 308. Proximal end 306 preferably includes a hub. Body 302 includes a lumen (not illustrated) extending between hub 312 and the closed distal end 308. Body 302 includes a second hub 312 and a side opening 316 to convey a perfusate between side opening 316 and hub 312. It will be appreciated that the reference to "side" is intended for easy reference and not limiting based on a particular position or orientation of the catheter in use

As shown in Figs. 9 and 10, distal end 308 preferably includes an atraumatic tip 322 preferably configured as a j-tip or hook.

As shown in Figs. 9 and 10, body 302 preferably includes an expandable support structure 330. Support structure 330 is preferably adapted to support and or dilate a blood vessel into which catheter 300 is inserted. In particular, support structure 330 is beneficial in supporting the wall of the blood vessel against collapse, especially when a negative pressure is present, thereby maintaining patency of the vessel. Support structure 330 preferably includes a plurality of trusses 332 equally circumferentially spaced about the mid-longitudinal axis of body 302. Each of the trusses has a first end 334 and a second end 336 connected at different points along the length of body 302. Preferably, first end 334 is connected to body 302 distally of side opening 316, while the second end 336 is connected proximally of side opening 316.

As shown in Figs. 9 and 10, support structure 330 is preferably shaped such that the distal portion 338 and the proximal portion 340 generally form an accentuated rise tempered by a plateau, said plateaus being joined by a straight segment 390 along a portion of body 302.

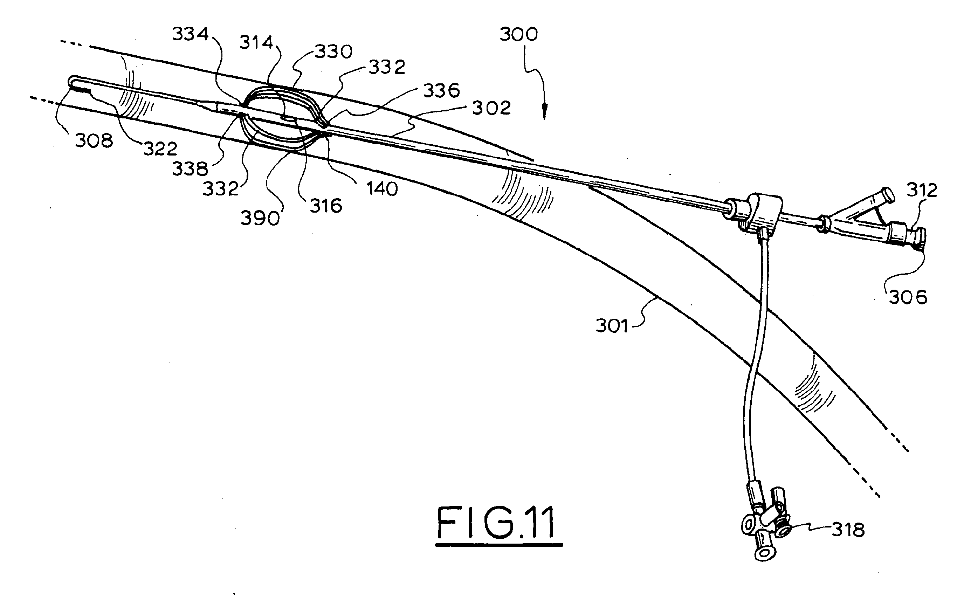

Fig. 11 shows a preferred embodiment of reverse catheter 300 having a body 302 in vessel 301.

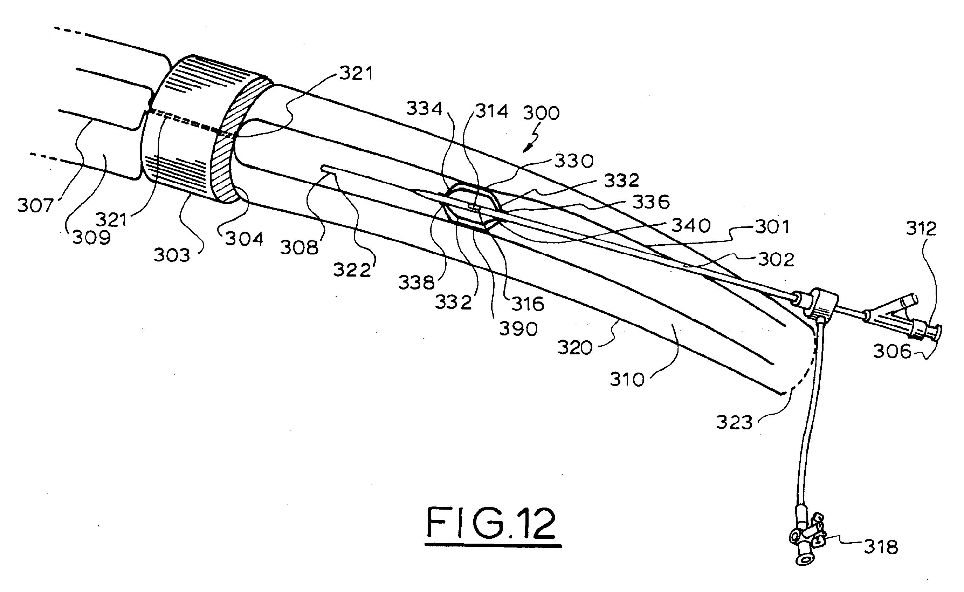

Figs. 12 show a preferred embodiment of a reverse catheter 300 having a body 302 where occlusion means is achieved by a constricting force applied at a defined point which can be achieved using a tourniquet 303 applied to the external surface of a limb 320 causing constriction of the external surface 304 bringing together the vessel walls 321. Vessel 307 distal to constriction remains patent. Vessel 307 and tissue 309 is substantially isolated from vessel 301 and tissue 310. End 323 is a defined end and is preferably a non-torso extremity or a portion of the non-torso extremity. The counterpart vessel is not depicted in Fig. 12. For example, if the vessel depicted is a vein, then the counterpart artery is not depicted, but would have a similar profile as would be appreciated.

A delivery device of the kind illustrated in Figs. 5 to 6A is suitable for delivering a perfusate (e.g. blood, oxygen, blood plus therapeutic agent) solution from the artificial flow path to targeted tissues. The perfusate may then be collected after passing through the target tissue using a venous collection device such as catheter 300 in Fig. 10. Collection solution can then be re-circulated through the targeted tissue with preferably re-oxygenated perfusate using a system such as system 10 in Fig. 4. Such a perfusion method is beneficial as it maximizes the efficiency of the therapeutic agent in the perfusate by eliminating break down in the liver and stomach, and also reduces or eliminates the toxicity issues associated with specific treatments reaching non-target tissue. Recirculation further improves the uptake of therapeutic agents by increasing the exposure time to the target tissue ultimately improving effectiveness of the treatment.

Having described the preferred components of 300, a preferred method of use will now be described with reference to Figs. 11 and 12, optionally Fig. 5. To perfuse an extremity, (the extremity including limbs, tails and heads) the medical practitioner inserts catheter (optionally catheter 200) into an artery in a retrograde direction relative to the blood flow and inserts catheter 300 into a vein in an antegrade direction relative to the blood flow in an undeployed state as shown in Figure 7A. Tourniquet 303 is inflated or tied to occlude the limbs vessels, in particular vessel 301. Catheter 300 is moved into its deployed state and engaged in its open conformation. Support structure may be deployed at the same time as tourniquet 303 or anytime after but before perfusion.

The perfusate is introduced into the blood stream through the hub of a catheter through the catheter's lumen (lumen of catheter 200) and its terminal opening into the blood vessel that is the artery. Blood flow is diverted from the vein through side opening 316, lumen 314. Thereafter the perfusate maybe circulated into a delivery catheter in the artery for re-use as desired or collect for later use or disposal. The perfusate being introduced into the delivery catheter may be replenished as needed and optionally supplemented by previously circulated perfusate. For example system 10 shown in Fig. 4 may be used in performing the above method. Other exemplary circuits are described in international application number PCT/US2007/0163669 (Publication No. WO 2008/011100).

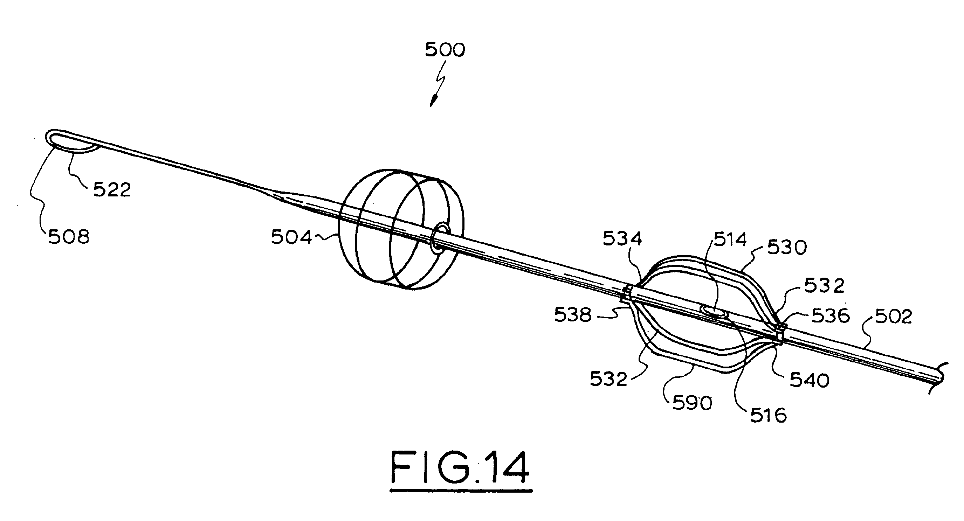

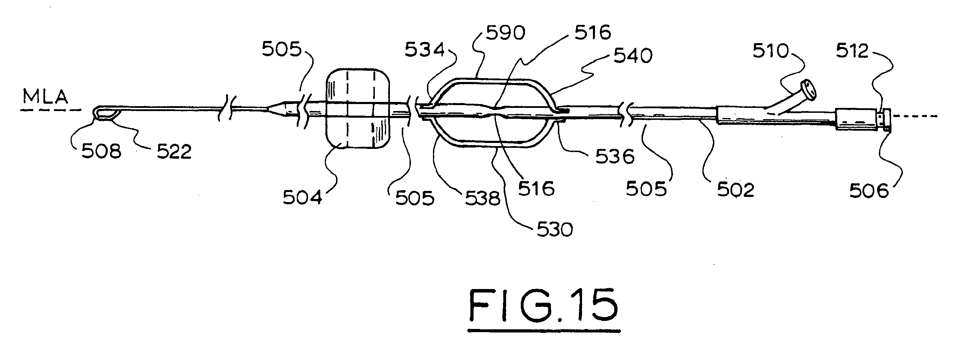

As shown in Figs. 8 and 8A, the catheters are preferably positioned at similar points in the respective artery and vein. It will be appreciated that positioning may be varied. It will be appreciated that catheter 100 and 200 as set out in Figs. 8 and 8A can be used in conjunction with tourniquet 303, such that tourniquet 303 is proximal. Alternatively, catheter 100 can be replaced with catheter 300 of Figs. 9 to 12 or catheter 500 of Figs. 14 and 15. Catheter 200 can be replaced with a standard catheter such as catheter 700 as per Fig. 17A or Fig. 17B, or alternatively catheter 400 as per Fig. 13 in the same orientation as catheter 700 in Fig. 17A, and respective combinations thereof when proximal tourniquet 303 is used as the primary means of occlusion of tissue vessels, specifically arterial and venous vessels.

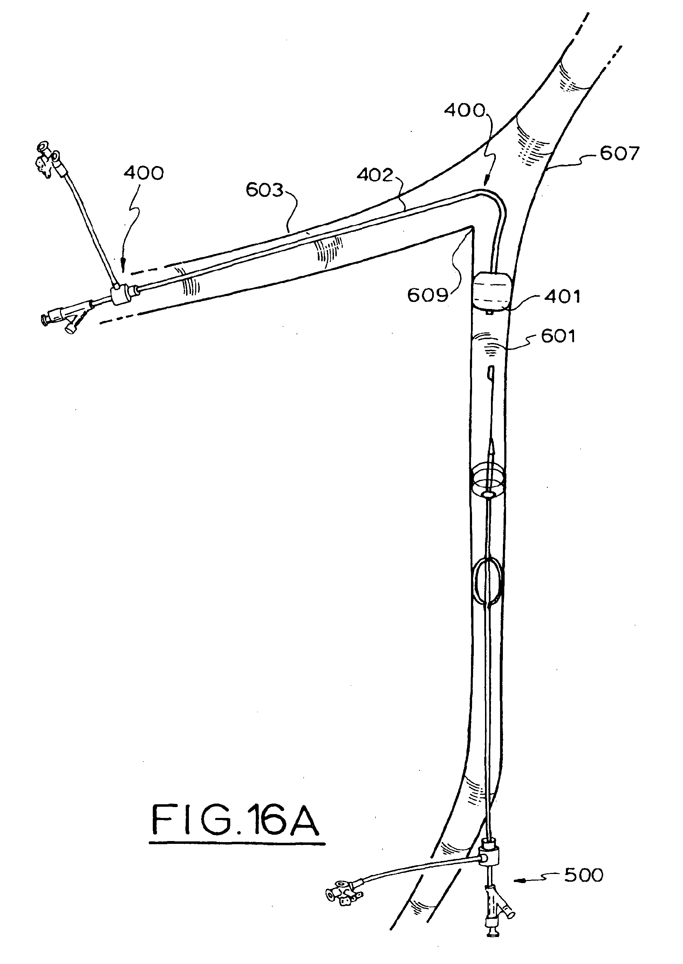

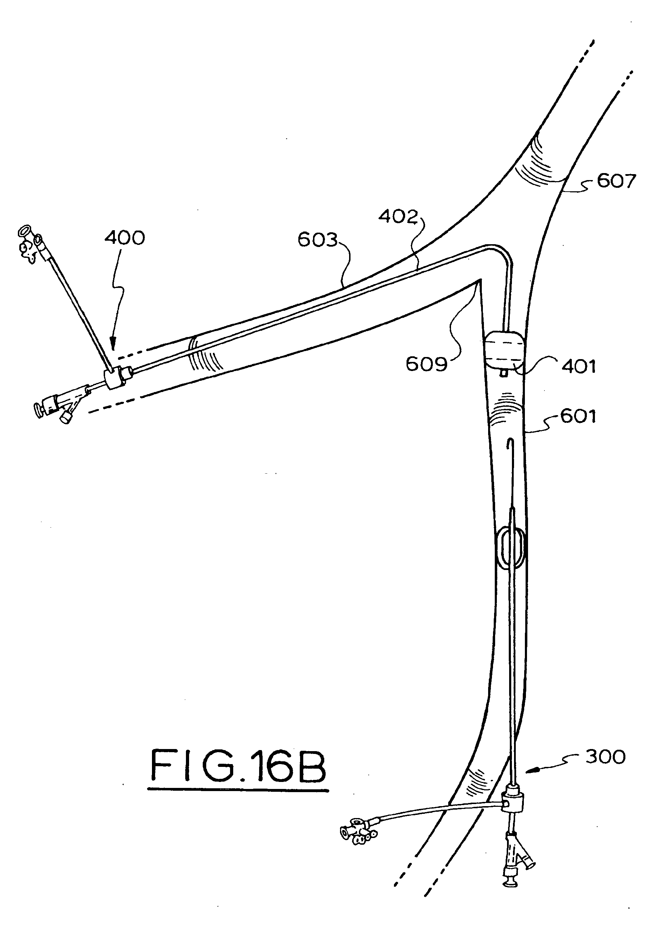

Figs. 13 to 17 show further preferred embodiments of the present invention. Referring to Fig. 13, a catheter 400 is shown in accordance with another preferred embodiment of the present invention. Catheter 400 is preferably configured for use as an occluding catheter as shown in Fig. 16A and Fig.16B. In Fig. 16B, catheter 400 may also have the additional and optional capability for use as an additional delivery catheter.

Referring to Figs. 14 and 15, a catheter 500 is shown in accordance with another preferred embodiment of the present invention. Catheter 500 has a body 502 and includes a proximal end 506, a preferably closed distal end 508 and a mid-longitudinal axis (MLA) running through proximal end 506 and distal end 508. Proximal end 506 preferably includes a hub 510 configured as an inflation hub for balloon 504. Body 502 includes a lumen (not illustrated) extending between hub 510 and balloon 504 to inflate and deflate balloon 504. Body 502 preferably includes a second hub 512 adapted to connect to a source of repository of perfusion fluid. Body 502 includes a lumen 514 extending between hub 512 and a side opening 516 to convey perfusate between side openings 516 and hub 512. It will be appreciated that the reference to "side" is intended for easy reference and not limiting based on a particular position or orientation of the catheter in use.

If desired, proximal end 506 may include hubs or ports for other uses such as described in relation to Fig. 3 above.

As shown in Figs. 14 and 15, distal end 508 preferably includes an atraumatic tip 522 preferably configured as a J-tip or hook or fish hook eye. It will be appreciated that tip 522 may be configured in a variety of ways without departing from the scope of the present invention.

Referring to Figs. 14 and 15, balloon 504 is preferably located closer to the distal end 508 than proximal end 506. It will be appreciated that distance 505 may be selected as desired depending upon the intended purpose. Balloon 504 is most preferably located between distal end 508 and the first end 534 of trusses 532 of support structure 530. As shown in Figs. 14 and 15, body 502 preferably includes an expandable support structure 530. Support structure 530 is preferably adapted to support and/or dilate a blood vessel into which catheter 500 is inserted. In particular, support structure 530 is beneficial in supporting the wall of the blood vessel against collapse especially when a negative pressure is present, thereby maintaining patency of the vessel. Support structure 530 preferably includes a plurality of trusses 532 equally circumferentially spaced about the mid-longitudinal axis of body 502. Each of the trusses has a first end 534 and a second end 536 connected at different points along the length of body 502. Preferably, first end 534 is connected to body 502 distally of side opening 516, while the second end 536 is connected proximally of side opening(s) 516. Support structure 530 is preferably in the range of 1mm to 30mm, more preferably 1mm to 12mm, most preferably approximately 7mm in diameter when in a deployed (expanded) position.

As shown in Figs. 14 and 15, support structure 530 is preferably shaped such that the distal portion 538 and the proximal portion 540 generally form an accentuated rise tempered by a plateau, the plateaus being joined by a straight segment 590 along a portion of body 502.

Fig. 16A illustrates reverse catheter 500 from Figs. 14 and 15 in use within vessel 601. In this preferred embodiment of reverse catheter 500, occlusion is achieved by balloon 401 of catheter 400 from Fig. 13 having body 402 that is placed in vessel 603 and passes over bifurcation 609 created by the meeting of vessels 602, 603 and 607, into vessel 601.

It will be appreciated that reverse catheter 100 from Figs. 1, 2 and 3 could be used in place of catheter 500 as shown in Fig. 16A.

Figure 16B illustrates reverse catheter 300 from Figs. 9 and 10 in vessel 601. In this preferred embodiment of reverse catheter 300, occlusion means is achieved by balloon 401 of catheter 400 having body 402 that is placed in vessel 603 and passes over bifurcation 609 created by the meeting of vessels 602, 603 and 607, into vessel 601. The counterpart vessel is not depicted in Figs. 16A and 16B. For example, if the vessel depicted is a vein, then the counterpart artery is not depicted, but would have a similar profile as would be appreciated.

A delivery device of the kind illustrated in Figs. 5 to 6A is suitable for delivering a perfusate (e.g., blood, oxygen, blood plus therapeutic agent) solution from the artificial flow path to targeted tissues in the orientation of Fig. 8A. Alternatively, the delivery device may be a delivery catheter without balloon placed in the orientation of Fig. 17. The perfusate may then be collected after passing through the target tissue using a collection device such as catheter 500 in Figs. 14 and 15 and as demonstrated in Figs. 16A and 16B. Collection solution can then be re-circulated through the targeted tissue with preferably re-oxygenated perfusate using a system such as system 10 in Fig. 4. Such a perfusion method is beneficial as it maximizes the efficiency of the therapeutic agent in the perfusate by eliminating break down in the liver and stomach, and also reduces or eliminates the toxicity issues associated with specific treatments reaching non-target tissue. Recirculation further improves the uptake of therapeutic agents by increasing the exposure time to the target tissue ultimately improving effectiveness of the treatment.

Having described the preferred components of catheter 400 and catheter 500, a preferred method of use will now be described with reference to Figs. 13 to 17. To perfuse an extremity, (the extremity including, e.g., limbs, tails and heads) the medical practitioner inserts a delivery catheter 700 (Fig. 17) into an artery in a retrograde direction relative to the blood flow contralaterally and manoeuvres it such that its final orientation is the antegrade direction relative to blood flow in the opposite artery. Alternatively the medical practitioner inserts a delivery catheter 700 into the target artery in a retrograde direction relative to the blood flow, with the final orientation being in the antegrade direction relative to blood flow or alternatively in the retrograde direction to the blood flow. Alternatively, delivery catheter 200 is inserted in a retrograde direction to flow in the target artery.

The medical practitioner then inserts catheter 100 or catheter 300 (as shown in Fig. 16B) or catheter 500 (Fig. 16A) in antegrade direction relative to the blood flow (in the vein) in an undeployed state as shown in Fig. 7A. The medical practitioner then inserts catheter 400 in the contralateral vein in an antegrade direction relative to the blood flow and manoeuvres it such that its final orientation is the retrograde direction relative to blood flow in the opposite vein as depicted in Fig. 16A and Fig. 16B.

Alternatively or in conjunction with catheter 400, tourniquet 303 is inflated or tied to occlude the limbs vessels, in particular vessel 301. Catheter 100, 300 or 500 is moved into its deployed state and engaged in its open conformation. The support structure may be deployed at the same time as catheter 400 or tourniquet 303 or anytime after, but preferably before perfusion.

The perfusate is introduced into the blood stream through the hub of a catheter through the catheter's lumen 414 or 714 respectively and its terminal opening into the blood vessel that is the artery. Blood flow is diverted from the vein through side opening 116, 316 or 516, respectively, into lumen 114, 314 or 514, respectively. Thereafter the perfusate may be circulated into a delivery catheter in the artery for re-use as desired or collected for later use or disposal. The perfusate being introduced into the delivery catheter may be replenished as needed and optionally supplemented by previously circulated perfusate. For example, system 10 shown in Fig. 4 may be used in performing the above method. Other exemplary circuits are described in international application number PCT/US2007/0163669 (Publication No. WO 2008/011100), the entire contents of which is hereby incorporated by reference herein. It will be appreciated that the embodiment described herein may be modified or used as desired and described in U.S. Application No. 61/087,177, titled "Reverse Catheter".

As shown in Figs. 16A, 16B, 17A and 17B, the catheters are preferably positioned at similar points in the respective artery and vein. It will be appreciated that positioning may be varied. It will be appreciated that catheters 100 and 200, as set out in Figs. 8 and 8A, catheter 500 and 400 as set out in Fig. 16A, catheter 300 and 400 as set out in Fig. 16B, and catheters 700 and 100 can be used in conjunction with tourniquet 303, such that tourniquet 303 is proximal. Alternatively, catheter 100 can be replaced with catheter 300 or catheter 500; and catheter 400 can be replaced with catheter 200 or a standard catheter, and respective combinations thereof, when proximal tourniquet 303 is used as the primary means of occlusion of tissue vessels, specifically arterial and venous vessels.

The foregoing description is by way of example only, and may be varied considerably without departing from the scope of the present invention. For example only, the catheter may include an open distal end in communication with the lumen conveying the perfusate, or in communication with a third lumen. The third lumen may be independent of the other lumens and adapted to receive therethrough a guide wire or other introducing instrument. One or more of the lumens may be concentric relative to the mid-longitudinal axis, or in a side-by-side relationship. One or more of the lumens may include one or more valves to assist in the control of the conveyance of the perfusate, and/or control of blood flow through the catheter. For example, where the catheter is adapted for use with a guide wire, a one-way valve may be included in the lumen containing the guide wire so that the valve may seal around the wire when the balloon is inflated (and before the pump is turned on).

In environments where a higher negative pressure might be needed, the catheter may be braded to enhance the structural integrity of the catheter.

The number, placement, shape and size of the side opening may be varied as desired. For example, side openings may be arranged equally spaced circumferentially around the mid-longitudinal axis of the body. Rows of openings may be arranged along the length of the body with varying sizes. For example, larger sized openings may be oriented closer to the balloon, while smaller sized openings are oriented further away from the balloon. The side openings may be shaped in a number of ways, for example, circular, oval, slit, or a grid. In one preferred embodiment, the side openings may be formed as a circumferential grid extending into the funnel region of the proximal portion of the balloon. This configuration is advantageous when the catheter is used, for example, as a drainage catheter. The shape of the balloon assists in diverting the flow of blood into the catheter.

Support structure 130 may be omitted if desired (as shown for example in Figs. 5 to 6A), or shaped in a variety of ways. For example, the trusses may each have a free end so as to be deployable similar to an umbrella, but with the free end being configured to reduce trauma (for example, being partially coiled or curved). The number of trusses may vary, for example, two, three, four, five, six, or more.

The catheter is preferably sized and shaped for the purpose intended. For example, the catheter may have a diameter in the range, for example only, from 1 mm to 10mm.

The catheter may include an external sleeve (such as cover 160 shown in Figs. 7A to 7C) or an internal rod or sheath to deploy the support structure manually. Preferably the side opening is located within the region between the proximal portion of the balloon and an end of the support structure to minimise interference of the blood vessel wall with the movement of fluid into or out of the side opening. For example, the side opening may be located a distance from the proximal portion of the balloon that is no greater than the maximum length of the balloon along the body.

Tip 120 may be internally guideable, or may be replaced with a guide wire as needed for a given situation.

Other instruments may be used in conjunction with the catheter as desired. For example, the catheter may be configured for use with a guide wire as described above, and/or with visualising instruments.

The device and system of the present invention may be applied to a variety of areas of the body, for example only, the lower extremeties such as the femoral arteries and/or veins, organs such as the liver, and other areas as desired. The present invention may be adapted for use in a variety of organs or anatomical regions. Exemplary organs include the lung, liver, kidney, brain, intestine, testicle, ovary, spleen, stomach, prostate, and pancreas. The anatomical region may be a limb, the pelvis, the chest, the breast or the mesenteric system.

Exemplary veins may include, but are not limited to a left or right internal jugular vein, a hepatic vein, a renal vein, a cephalic vein, an inferior vesical vein, a pulmonary vein, an internal iliac vein, a portal vein, a splenic vein, a femoral vein, a saphenous vein, a subclavian vein, an intercostal vein, an axillary vessel, a left or right testicular vein, a left or right ovarian vein, a pulmonary vein or tributaries thereof.

Exemplary arteries may include, but are not limited to a left or right vertebral artery, a left or right internal carotid artery, an inferior vesicular artery, an internal iliac artery, a renal artery, a gastric artery, a splenic artery, a superior or inferior mesenteric artery, an internal iliac artery, an internal mammary artery, an intercostal artery, a right or left testicular artery, a left or right ovarian artery, a pulmonary artery or tributaries thereof.

The delivery catheter and the support catheter may be used in the same vessel, which is beneficial if the practitioner desires to treat just an arterial or venous system.

The method may further include the step of using an imaging technique to position the delivery catheter and/or collection catheter in the inflow and outflow vessels of the target region, the imaging technique including one or more of deployment of radiographic contrast, deployment of nuclear medical probes, deployment of in vivo probes sensitive to oxygen, hydrogen, pH or the like and deployment of labelled micro- or nano- particles. The imaging technique may be facilitated by including one or more materials in the tip of the catheter that are visible on the image produced by the imaging device. Ultrasound catheters may be used in lieu of, or in addition to the imaging techniques described above.

Embodiments of the present invention may be used in a range of blood vessels servicing different organs of the body including the lung, liver, kidney, brain, intestine, testicle, ovary, spleen, stomach, prostate and pancreas. The present invention may also be used in blood vessels servicing an entire anatomical region such as a limb, the pelvis, the chest, the breast and the mesenteric system. The skilled person will possess sufficient knowledge to decide the appropriate vessel or vessels to target according to which organ or anatomical region is to be catheterized. It is envisaged that the present invention has application beyond blood vessels, and may be used in other anatomical vessels or enclosed structures.

Where the organ is the liver, an arterial catheter may be placed in the hepatic artery and a venous catheter may be placed in the hepatic vein. This may be performed with a reduction of flow in the portal vein utilizing acute administration of, for example, Octreotide or Terlipressin.

Where the organ is the brain, arterial catheters may be placed in the left and right vertebral arteries, and also the left and right internal carotid arteries. Venous catheters may be placed in the left and right internal jugular veins.

Perfusion of the kidney may be performed by locating an arterial catheter in the renal artery. A venous catheter may be placed in the renal vein. Where the organ is the stomach, an arterial catheter may be placed in the gastric artery. A venous catheter may be placed in the portal vein or the hepatic veins.

Perfusion of the spleen may be achieved by placing an arterial catheter may in the splenic artery, and a venous catheter in the splenic vein, the portal vein, or the hepatic veins. To perfuse the intestines, an arterial catheter may be placed in the superior and/or inferior mesenteric artery. A venous catheter may be placed in the portal vein or hepatic veins.

Where the anatomical region is the pelvis, an arterial catheter may be placed in the internal iliac artery. Where the organ is a testicle or an ovary, an arterial catheter may be placed in the right or left testicular or ovarian artery. A venous catheter may be placed in the right or left testicular or ovarian vein. To perfuse the prostate, an arterial catheter may be placed in the inferior vesicle artery or the internal iliac artery. A venous catheter may be placed in the inferior vesical vein or the internal iliac vein.

Where the organ is a lung, an arterial catheter may be placed in the pulmonary artery. A venous catheter may be placed in one or both pulmonary veins. In one embodiment, selective delivery can be provided to one lung only, or only one lobe of a lung. Thus, the arterial catheter may be located in the relevant branch of the pulmonary artery and the venous catheter would be located in the relevant (i.e. upper or lower) pulmonary vein, depending on the lobe being treated. To perfuse the breast, an arterial catheter may be placed in the internal mammary artery and the intercostal arteries. A venous catheter may be placed in the intercostal veins.

It will be appreciated that other occlusion means may be used in addition to or in lieu of a balloon. For example, a suitably configured catheter may be inserted as described above after clamping one or more portions of the blood vessel instead of, or in addition to deploying a balloon. A tourniquet may be applied for occlusion in addition to or in lieu of a balloon as desired.

The features described with respect to one embodiment may be applied to other embodiments, or combined with or interchanged with the features other embodiments, as appropriate, without departing from the scope of the present invention.

The present invention in one or more preferred forms provides the advantages of being shorter and able to withstand higher flow rates than a standard length catheter. Therefore the catheter diameter may be reduced to be less invasive. A catheter of reduced length is easier to insert and control when compared to conventional length catheters. A further advantage is that in a preferred method of the present invention, for example, in delivering perfusate, the need to cross the aortic bifurcation is eliminated, reducing the length of the procedure.

In a preferred form, the supporting structure is configured to substantially prevent collapse of the blood vessel or other tissue under negative pressure conditions.

The maintenance of the vessel enables an artificial circuit to be established, which may be automated with the use of one or more appropriately placed detectors and pumps.

The system described has wide applicability and may be adapted for use in animals other than humans, such as horses, dogs, cats, bulls, sheep and other sporting animals, domestic pets and/or livestock.

## Claims

1. A catheter (100) for conducting a fluid into or out of a blood vessel, said catheter comprising:
a body (102) having a proximal end (106), a closed distal end (108) and a mid-longitudinal axis; and
a balloon (104) attached to said body, said balloon having a proximal portion (124) and a distal portion (126), said body having a first lumen in communication with said balloon and a second lumen (114) adapted to convey a fluid to or from the blood vessel through a side opening (116) in said body, said side opening being located rearwardly adjacent said proximal portion of said balloon;
wherein the catheter further comprises a plurality of trusses (132) adapted to support the blood vessel, each of said trusses having a first end (134) and a second end (136) connected at different points along the length of said body 102, and wherein said trusses (132) extend over said balloon (104) along the length of said balloon, **characterized in that** said side opening (116) is located between said proximal portion (124) of said balloon (104) and one of the ends (134, 136) of said trusses (132).

2. The catheter (100) of claim 1, wherein said first and second (114) lumens are oriented concentric relative to one another.

3. The catheter (100) of claim 1, wherein said first and second 114) lumens are oriented in a side-by-side arrangement relative to one another.

4. The catheter (100) of any one of claims 1 to 3, wherein said side opening (116) is located a distance from said proximal portion (124) of the balloon (104) that is no greater than the maximum length of said balloon along said body (102).

## Patentansprüche

1. Katheter (100) zum Leiten eines Fluids in ein Blutgefäß hinein oder aus einem Blutgefäß heraus, wobei der Katheter Folgendes umfasst:
einen Körper (102) mit einem proximalen Ende (106), einem geschlossenen distalen Ende (108) und einer Mittenlängsachse und
einen Ballon (104), der an dem Körper angebracht ist und einen proximalen Abschnitt (124) und einen distalen Abschnitt (126) hat, wobei der Körper ein erstes Lumen, das mit dem Ballon in Verbindung steht, und ein zweites Lumen (114) hat, das geeignet ist, ein Fluid durch eine Seitenöffnung (116) in dem Körper in ein Blutgefäß hinein- oder aus einem Blutgefäß herauszufördern, wobei die Seitenöffnung hinten in der Nähe des proximalen Abschnitts des Ballons angeordnet ist,
wobei der Katheter ferner mehrere Versteifungen (132) umfasst, die geeignet sind, das Blutgefäß zu stützen, wobei jede der Versteifungen ein erstes Ende (134) und ein zweites Ende (136) hat, die an verschiedenen Stellen entlang der Länge des Körpers (102) verbunden sind, und wobei sich die Versteifungen (132) entlang der Länge des Ballons (104) über diesen erstrecken, **dadurch gekennzeichnet, dass** die Seitenöffnung (116) zwischen dem proximalen Abschnitt (124) des Ballons (104) und einem der Enden (134, 136) der Versteifungen (132) angeordnet ist.

2. Katheter (100) nach Anspruch 1, wobei das erste und das zweite Lumen (114) zueinander konzentrisch ausgerichtet sind.

3. Katheter (100) nach Anspruch 1, wobei das erste und das zweite Lumen (114) nebeneinander ausgerichtet sind.

4. Katheter (100) nach einem der Ansprüche 1 bis 3, wobei die Seitenöffnung (116) in einem Abstand von dem proximalen Abschnitt (124) des Ballons (104) angeordnet ist, der nicht größer als die maximale Länge des Ballons entlang dem Körper (102) ist.

## Revendications

1. Cathéter (100) pour conduire un liquide dans ou hors d'un vaisseau sanguin, ledit cathéter comprenant :
un corps (102) ayant une extrémité proximale (106), une extrémité distale fermée (108) et un axe longitudinal moyen ; et
un ballonnet (104) fixé audit corps, ledit ballonnet ayant une partie proximale (124) et une partie distale (126), ledit corps ayant une première lumière en communication avec ledit ballonnet et une seconde lumière (114) adaptée pour transporter un liquide vers ou depuis le vaisseau sanguin au travers d'une ouverture latérale (116) dans ledit corps, ladite ouverture latérale étant située vers l'arrière en position adjacente à ladite partie proximale dudit ballonnet ;
où le cathéter comprend en outre une pluralité de renforts (132) adaptés pour soutenir le vaisseau sanguin, chacun desdits renforts ayant une première extrémité (134) et une seconde extrémité (136) reliées à des points différents sur la longueur dudit corps (102), et où lesdits renforts (132) s'étendent sur ledit ballonnet (104) sur la longueur dudit ballonnet, **caractérisé en ce que** ladite ouverture latérale (116) est située entre ladite partie proximale (124) dudit ballonnet (104) et l'une des extrémités (134, 136) desdits renforts (132).

2. Cathéter (100) selon la revendication 1, où lesdites première et seconde lumières (114) sont orientées de manière concentrique l'une par rapport à l'autre.

3. Cathéter (100) selon la revendication 1, où lesdites première et seconde lumières (114) sont orientées dans un agencement côte à côte l'une par rapport à l'autre.

4. Cathéter (100) selon l'une quelconque des revendications 1 à 3, où ladite ouverture latérale (116) est située à une distance de ladite partie proximale (124) du ballonnet (104) qui n'est pas supérieure à la longueur maximale dudit ballonnet le long dudit corps (102).
